(19) Europäisches Patentamt
European Patent Office
Office européen des brevets

(11) Veröffentlichungsnummer: **0 104 445**
**A2**

# (12) EUROPÄISCHE PATENTANMELDUNG

(21) Anmeldenummer: 83108375.3

(22) Anmeldetag: 25.08.83

(51) Int. Cl.³: C 07 D 413/14
C 07 D 491/056, A 61 K 31/505

(30) Priorität: 25.09.82 DE 3235565

(43) Veröffentlichungstag der Anmeldung:
04.04.84 Patentblatt 84/14

(84) Benannte Vertragsstaaten:
AT BE CH DE FR GB IT LI LU NL SE

(71) Anmelder: BOEHRINGER INGELHEIM KG
ZA Patente
D-6507 Ingelheim am Rhein(DE)

(72) Erfinder: Mentrup, Anton, Dr.
Steinernstrasse 25
D-6503 Mainz-Kastel(DE)

(72) Erfinder: Renth, Ernst-Otto, Dr.
Frankenstrasse 11
D-6507 Ingelheim am Rhein(DE)

(72) Erfinder: Schromm, Kurt, Dr.
In der Dörrwiese 35
D-6507 Ingelheim am Rhein(DE)

(72) Erfinder: Hoefke, Wolfgang, Dr.
Rosselstrasse 21
D-6200 Wiesbaden(DE)

(72) Erfinder: Gaida, Wolfram, Dr.
Paul-Clemen-Strasse 14
D-6507 Ingelheim am Rhein(DE)

(54) **Piperidinochinoxalin-Derivate, ihre Herstellung und Verwendung.**

(57) Zusammenfassung es werden neue Verbindungen der Formel

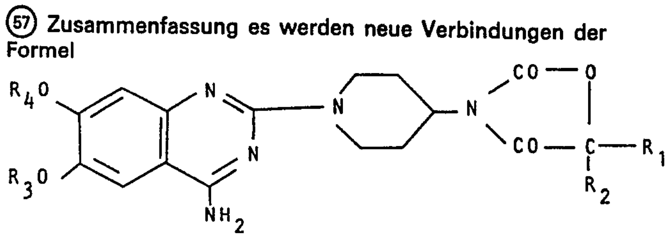

(I)

beschrieben, ihre Herstellung und ihre Verwendung als Arzneistoffe.

EP 0 104 445 A2

BEZEICHNUNG GEÄNDERT
siehe Titelseite





Die Erfindung betrifft neue Piperidinderivate, ihre Herstellung nach an sich bekannten Verfahren und ihre Verwendung zur Behandlung von Herz/Kreislauferkrankungen.

Die neuen Verbindungen entsprechen der Formel

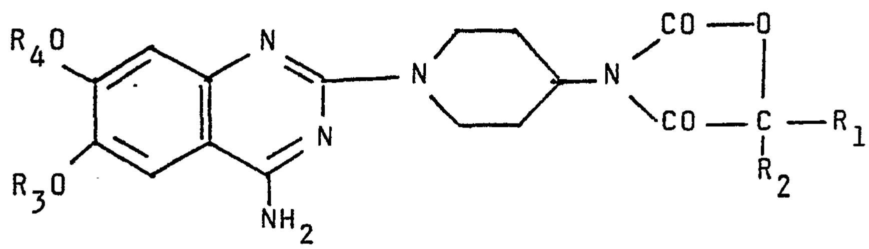

(I)

in der (wie auch im folgenden)

$R_1$ und $R_2$, die gleich oder verschieden sein können, für Wasserstoff, $C_1$-$C_8$-Alkyl oder Arylmethyl, $R_1$ und $R_2$ gemeinsam auch für $-(CH_2)_4-$ oder $-(CH_2)_5-$,

$R_3$ und $R_4$, die gleich oder verschieden sein können, für Wasserstoff, $C_1$-$C_4$-Alkyl oder Arylmethyl, $R_3$ und $R_4$ gemeinsam auch für $-CH_2-$ oder $-CH_2-CH_2-$ stehen können.

Die Reste $R_1$ und $R_2$ haben bevorzugt Wasserstoff oder $C_1$-$C_4$-Alkyl, $R_3$ und $R_4$ bevorzugt Methyl. Die Alkylreste können geradkettig oder verzweigt sein. Unter "Arylmethyl" ist vor allem Benzyl oder substituiertes Benzyl zu verstehen.

Die Verbindungen der Formel I können in freier Form oder als Salze mit Säuren vorliegen, wobei sie je nach der

Substitution ($R_1$ und $R_2$ gleich oder verschieden) ggf. auch Racemate bzw. Stereoisomerengemische oder reine Enantiomere darstellen.

Zur Herstellung der neuen Verbindungen dienen an sich bekannte Verfahren.

1. Man setzt ein 4-Aminochinoxalin der Formel

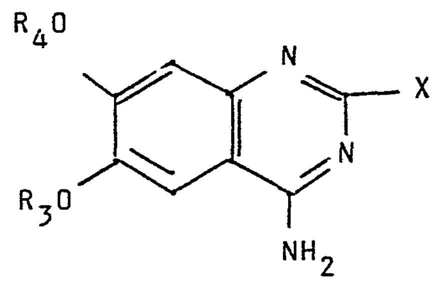

(II),

worin X für Chlor, Brom oder $C_1$-$C_4$-Alkylthio steht, mit einem Piperidinderivat der Formel

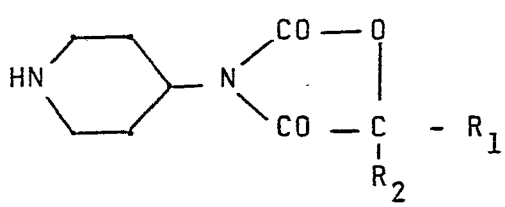

(III)

um.

Die Reaktion wird in Gegenwart von säurebindenden Mitteln, wie Natriumcarbonat, Kaliumcarbonat oder Aminen, etwa Tripropylamin, durchgeführt oder es werden 2 Äquivalente bzw. ein Überschuß von III eingesetzt.

Die Umsetzung erfolgt vorzugsweise in der Wärme, zweckmäßig in einem inerten Lösungsmittel bei Rückflußtemperatur.

Die Ausgangsstoffe der Formel II sind bekannt oder können nach üblichen Verfahren gewonnen werden. Die Ausgangsstoffe der Formel III sind z.B. nach folgendem Reaktionsschema erhältlich:

$HO-CR_1R_2-COOC_4H_9$ + $H_2N-CO-NH_2$

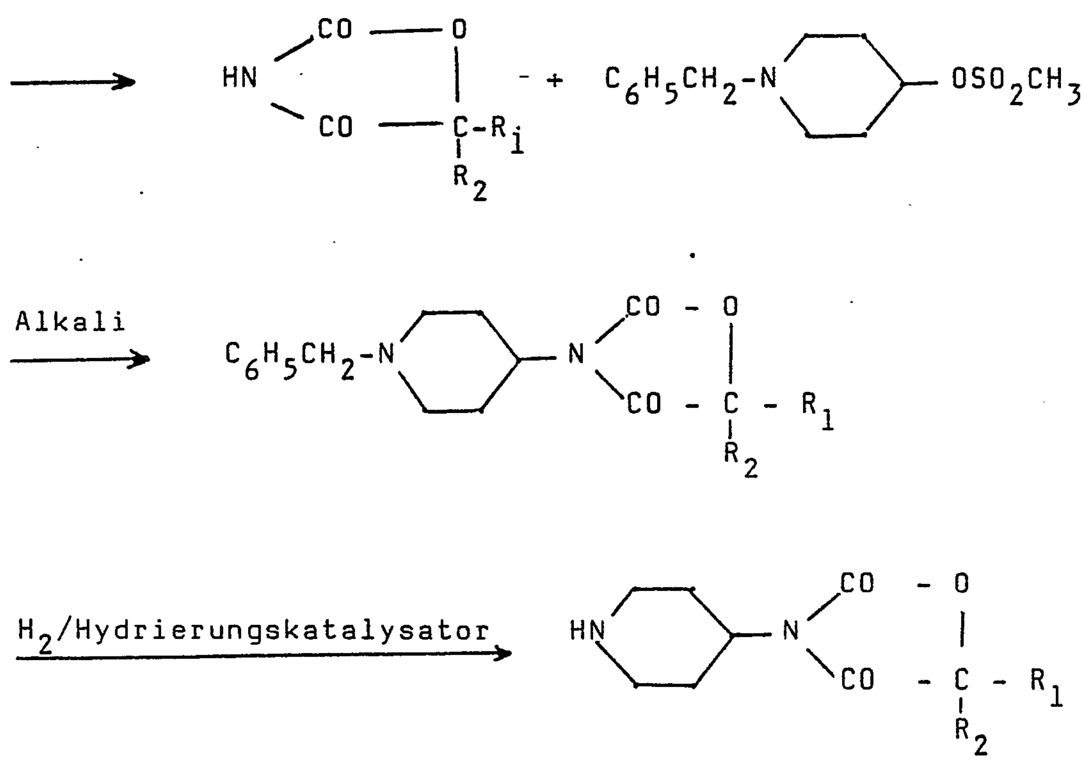


(III)

2. Man setzt eine Verbindung der Formel

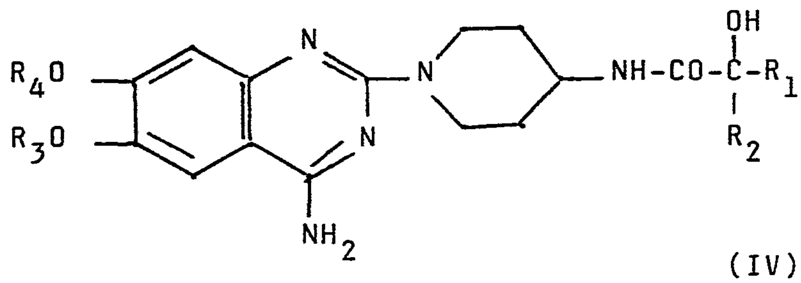

(IV),

worin
$R_1$, $R_2$, $R_3$ und $R_4$ die obige Bedeutung haben, mit einem Dialkylcarbonat der Formel

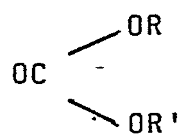

(V),

worin R und R', die gleich oder verschieden sein können, für gegebenenfalls verzweigte $C_1$-$C_6$-Alkylreste stehen, um.

Die Umsetzung erfolgt bei erhöhter Temperatur unter Zusatz einer geringen Menge Natriumhydrid und eines niederen Alkohols, etwa Ethanol. Das Dialkylcarbonat wird zweckmäßig im Überschuß verwendet und dient gleichzeitig als Lösungsmittel, so daß ein zusätzliches inertes Lösungsmittel im allgemeinen entbehrlich ist.

Die Ausgangsstoffe sind bekannt oder können in an sich bekannter Weise hergestellt werden.

Die Verbindungen der Formel IV können beispielsweise erhalten werden, indem man eine Verbindung der Formel

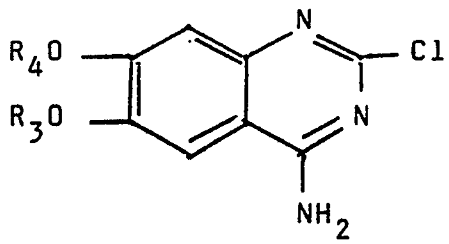

($R_3$ und $R_4$ in der obigen Bedeutung)

mit einer Verbindung der Formel

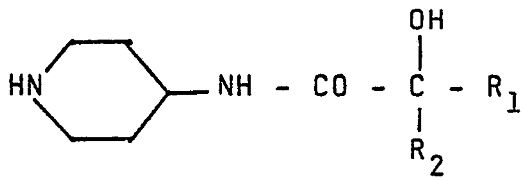

umsetzt.

3. Man setzt ein Carbamat der Formel

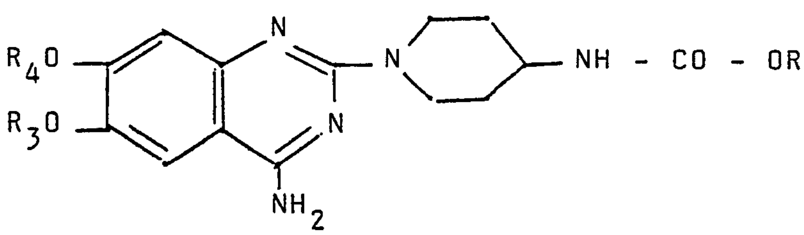

(VI),

worin R, $R_3$ und $R_4$ die obige Bedeutung haben, mit einem Hydroxicarbonsäureester der Formel

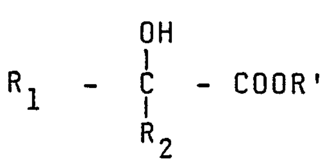

(VII)

um, worin R', $R_1$ und $R_2$ die obige Bedeutung haben.

Die Umsetzung erfolgt in der Wärme in einem ausreichend hoch siedenden inerten Lösungsmittel, z.B. Ethylenglykoldimethylether, in Gegenwart einer geringen Menge Natriumhydrid.

Die Ausgangsstoffe sind bekannt oder können nach an sich bekannten Verfahren hergestellt werden.

4. Man setzt eine Verbindung der Formel

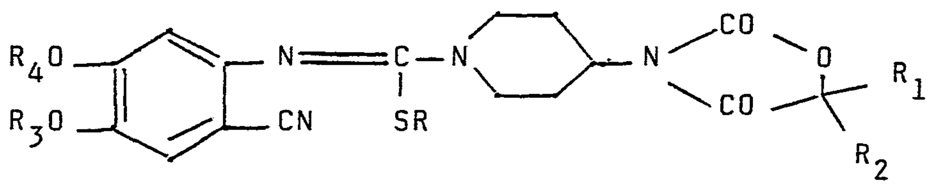

(VIII),

in der R, $R_1$, $R_2$, $R_3$ und $R_4$ die obige Bedeutung haben, mit Ammoniumchlorid und Formamid in der Wärme um.

Die Reaktion erfolgt vorzugsweise in einem Überschuß von Formamid, so daß ein zusätzliches inertes Lösungsmittel nicht erforderlich ist.

Die Ausgangsstoffe der Formel VIII können in an sich bekannter Weise erhalten werden, beispielsweise nach dem Schema

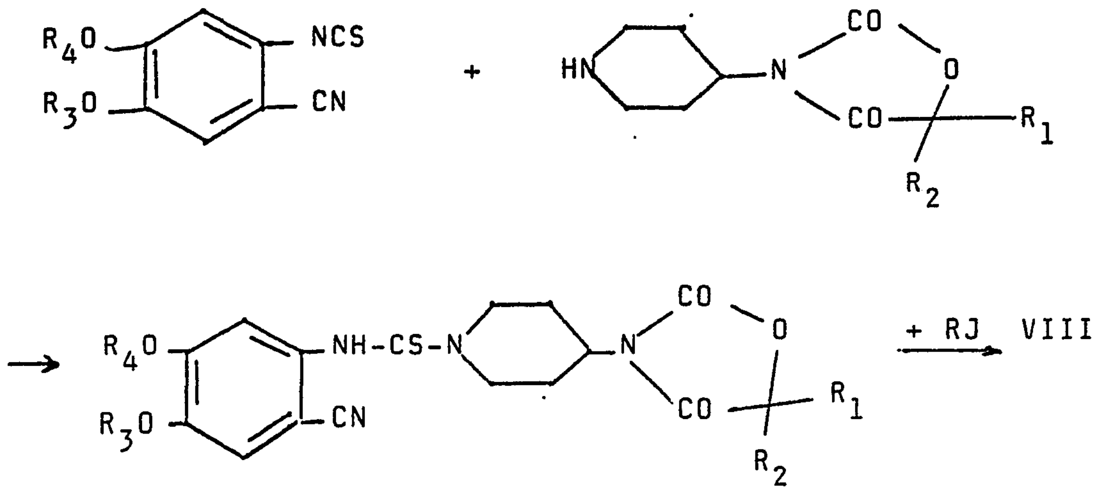


Soweit in den Verbindungen der Formel I $R_1$ und $R_2$ unterschiedlich sind, können zunächst erhaltene Enantiomerengemische nach üblichen Methoden aufgetrennt werden. Man kann aber auch optisch aktive Verbindungen der Formel III als Ausgangsstoffe einsetzen.

Die neuen Verbindungen sind als Arzneistoffe verwendbar. Sie zeigen eine ausgeprägte gefäßerweiternde Wirkung und stellen wertvolle Mittel zur Therapie von Herz/Kreislauferkrankungen dar. Ganz besonders geeignet sind sie für die Therapie des Bluthochdrucks.

Die überraschend starke und langanhaltende blutdrucksenkende Wirkung zeigt sich z.B. tierexperimentell an wachen SH-Ratten nach oraler Gabe oder an narkotisierten Kaninchen nach intravenöser Gabe. Eine ausgeprägte $\alpha$-adrenolytische Wirkung kann an der Rattensamenblase nachgewiesen werden. Im Vergleich zu den bekannten $\alpha$-Adrenolytika Regitin und Prazosin zeigen die erfindungsgemäßen Verbindungen eine überlegene Wirksamkeit.

Die lange anhaltende blutdrucksenkende Wirkung wurde z.B. an Ratten geprüft. Bei oraler Gabe von 10 mg/kg der Verbindungen Nr. 3, 4, 6 und 7 (Tabelle I) beispielsweise wurde an fünf aufeinanderfolgenden Tagen jeweils nach 6 Stunden noch eine Blutdrucksenkung von mehr als 20 mm Hg ermittelt.

Für therapeutische Zwecke werden die Verbindungen der Formel I oder ihre geeigneten Salze mit gebräuchlichen Hilfs- und/oder Trägerstoffen zu üblichen galenischen Zubereitungen verarbeitet, z.B. zu Tabletten, Dragées, Kapseln, Lösungen, Suppositorien. Geeignete Salze sind beispielsweise die Chloride, Methansulfonate, Bromide, Sulfate, Formiate, Acetate, Succinate, Glykolate, Citrate, Maleinate, Nicotinate, Pamoate, Phenylacetate, Benzoate, Cyclohexylsulfaminate und Tosylate. Die Tabletten, Dragées und Kapseln enthalten jeweils zwischen 0,1 und 50 mg, vorzugsweise 0,5 bis 10 mg Wirkstoff.

Die erfindungsgemäßen Verbindungen eignen sich auch für die Anwendung in Kombination mit einem oder mehreren anderen Arzneistoffen. Besonders in Betracht kommen Kombinationen mit Herz/Kreislauf-wirksamen bzw. blutdrucksenkenden Stoffen. Zu nennen sind vor allem Diuretika, ß-Blocker, Vasodilatatoren, Sympathikolytika und Converting-Enzymblocker. Als Beispiele für Wirkstoffe der genannten Kategorien seien angeführt:

| | |
|---|---|
| Acebutolol | Labetalol |
| Allopurinol | Metolazon |
| α-Methyldopa | Metoprolol |
| Alprenolol | Minoxidil |
| Atenolol | Nadolol |
| Bumetanid | Natriumnitroprussid |
| Capopril | Oxprenolol |
| Chlorthalidon | Phentolamin |
| Clonidin | Pindolol |
| Debrisoquin | Prazosin |
| Diazoxid | Propanolol |
| Dihydralazin | Reserpin |
| Etacrinsäure | Ro 12-4713 Larovasin |
| Furosemid | Sotalol |
| Guanfacin | Tienilsäure |
| Hydrochlorothiazid | Timolol |
| Indapamid | Verapamil |

Formulierungsbeispiele:

A. Tabletten

Zusammensetzung:

| | |
|---|---|
| Wirkstoff nach Formel I | 1 mg. |
| kolloidale Kieselsäure | 10 mg |
| Kartoffelstärke | 60 mg |
| Milchzucker | 117 mg |
| Polyvinylpyrrolidon | 6 mg |
| Natrium-Celluloseglykolat | 4 mg |
| Magnesiumstearat | 2 mg |
| | 200 mg |

Die Bestandteile werden in üblicher Weise zu Tabletten verarbeitet.

B. Kapseln

Zusammensetzung:

| | |
|---|---|
| N-[1-(4-Amino-6,7-dimethoxi-2-chinazolinyl)-4-piperidyl]-5-isopropyl-oxazolidin-2,4-dion-methansulfonat | 5 mg |
| Maisstärke | 295 mg |
| | 300 mg |

Die feinteiligen Bestandteile werden entsprechend dem angegebenen Mengenverhältnis gut vermischt und in 300 mg-Portionen in Gelatine-Steckkapseln abgefüllt.

Die erfindungsgemäßen Herstellungsverfahren sollen durch die nachstehenden Beispiele näher erläutert werden.

Beispiel 1

N-[1-(4-Amino-6,7-dimethoxi-2-chinazolinyl)-4-piperidyl]-oxazolidin-2,4-dion

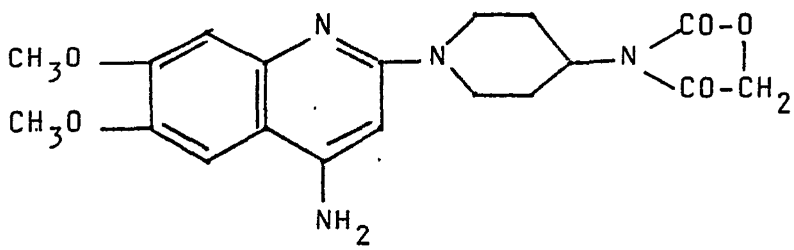

2,4 g 2-Chlor-4-amino-6,7-dimethoxichinazolin und 2,32 g N-(4-Piperidyl)-oxazolidin-2,4-dion Hydrochlorid werden mit 3 g Tri-n-propylamin in 50 ml Ethylglykol 2 Stunden unter Rückfluß gekocht. Das in der Kälte ausgeschiedene Produkt läßt sich nach Zugabe von 50 ml Äther als Hydrochlorid in einer Ausbeute von 4 g isolieren. Zur Überführung in die Base wird das Hydrochlorid in 25 ml Wasser suspendiert und mit 15 ml wässrigem Ammoniak versetzt. Die in einer Ausbeute von 95,5 % isolierte Base hat nach dem Umkristallisieren aus Methanol den Schmp. 230°C.

Aus der in Methanol heiß gelösten Base kristallisiert nach Zugabe der berechneten Menge Methansulfonsäure das Methansulfonat (Fp. 307°C) aus.

Das aus Ausgangsmaterial verwendete N-(4-Piperidyl)-oxazolidin-2,4-dion wird hergestellt durch eine Hydrierung von 12 g N-[4-(1-Benzyl-piperidyl]-oxazolidin-2,4-dion in 400 ml Methanol nach Zugabe von 12 ml einer 13,6 %igen methanolischen Salzsäurelösung in Gegenwart von Palladiumkohle als Katalysator. Die Ausbeute der als Hydrochlorid isolierten Substanz beträgt 72 % (Fp. 287°C).

Das als Ausgangsmaterial verwendete N-[4-(1-Benzylpiperidyl]-oxazolidin-2,4-dion wird erhalten durch die Zugabe von 30 g 4-Methansulfonyloxy-1-benzylpiperidin zu einer Lösung von 10,1 g Oxazolidin-2,4-dion und 4,2 g Natriumhydrid in 120 ml Hexametapol. Nach einer dreistündigen Reaktion wird das Produkt in einer Ausbeute von 45 % als Maleinat (Fp. 206°C) isoliert.

Analog Beispiel 1 werden die folgenden Substanzen der Formel I erhalten:

Tabelle I

| Nr. | $R_1$ | $R_2$ | $R_3$ | $R_4$ | Fp [° C] Base | Salz |
|---|---|---|---|---|---|---|
| 1 | $CH_3$ | $CH_3$ | $CH_3$ | $CH_3$ | 223 | 293 ($CH_3SO_3$-H) |
| 2 | $CH_3$ | H | $CH_3$ | $CH_3$ | | 280 (HCl) |
| 3 | $CH_3$ | $C_2H_5$ | $CH_3$ | $CH_3$ | 135 | 288 ($CH_3SO_3H$) |
| 4 | n-$C_4H_9$ | H | $CH_3$ | $CH_3$ | 80 | 230 ($CH_3SO_3H$) |
| 5 | $-(CH_2)_4-$ | | $CH_3$ | $CH_3$ | 140 | 295 ($CH_3SO_3H$) |
| 6 | $CH(CH_3)_2$ | H | $CH_3$ | $CH_3$ | | 272 ($CH_3SO_3H$) |
| 7 | $C_2H_5$ | H | $CH_3$ | $CH_3$ | | 298 ($CH_3SO_3H$) |
| 8 | n-$C_3H_7$ | H | $CH_3$ | $CH_3$ | | 243 ($CH_3SO_3H$) |
| 9 | $C_6H_5-CH_2$ | H | $CH_3$ | $CH_3$ | | 284 (HCl) |

Beispiel 2

a) 2-/4-(3-Methyl-2-hydroxi)-butyramido7-piperid-1-yl-4-amino-6,7-dimethoxi-chinazolin

Ein Gemisch aus 2,9 g (0,012 mol) 2-Chlor-4-amino-6,7-dimethoxi-chinazolin(1), 2,4 g (0,012 mol) 4-(2-Hydroxi-3-methyl-butyramido)-piperidin, 2,3 ml (0,012 mol) Tri-n-propyl-amin und 40 ml Ethylglykol wird 2 Stunden unter Rückfluß gekocht. Man dampft ein und nimmt den öligen Rückstand in halbkonz. Ammoniakwasser und Essigester auf. Die Essigester-Phase wird getrocknet und eingedampft. Isoliert werden 4,6 g der Titelverbindung.

b) 1-/1-(4-Amino-6,7-dimethoxi-2-chinazolinyl)-4-piperidyl7-5-isopropyl-2,4-oxazolidindion-methansulfonat

Eine Suspension aus 2,0 g (0,005 mol) der Verbindung (a), 4 ml (0,033 mol) Diethylcarbonat, 40 mg (0,001 mol) Natriumhydrid-Dispersion (57,5 %ig) und 0,5 ml Ethanol wird über 5 Stunden bei einer Außentemperatur von 170°C zur Reaktion gebracht. Dabei destilliert das Ethanol langsam ab. Das Gemisch kühlt man auf Raumtemperatur ab und versetzt es mit Eiswasser. Nach dem Ausschütteln mit Essigester wird nochmals mit Wasser gewaschen, getrocknet und eingedampft. Der ölige Rückstand wird mit Methansulfonsäure versetzt. Das Salz hat nach dem Umkristallisieren den Schmelzpunkt 272°C.

Analog Beispiel 2 werden die folgenden Substanzen der Formel I erhalten:

Tabelle II

| Nr. | $R_1$ | $R_2$ | $R_3$ | $R_4$ | Fp [° C] Base | Fp [° C] Salz |
|---|---|---|---|---|---|---|
| 1 | $CH_3$ | $CH_3$ | $CH_3$ | $CH_3$ | 223 | 293 ($CH_3SO_3$-H) |
| 2 | $CH_3$ | H | $CH_3$ | $CH_3$ | | 280 (HCl) |
| 3 | $CH_3$ | $C_2H_5$ | $CH_3$ | $CH_3$ | 135 | 288 ($CH_3SO_3H$) |
| 4 | n-$C_4H_9$ | H | $CH_3$ | $CH_3$ | 80 | 230 ($CH_3SO_3H$) |
| 5 | $-(CH_2)_4-$ | | $CH_3$ | $CH_3$ | 140 | 295 ($CH_3SO_3H$) |
| 6 | H | H | $CH_3$ | $CH_3$ | | 307 ($CH_3SO_3H$) |
| 7 | $C_2H_5$ | H | $CH_3$ | $CH_3$ | | 298 ($CH_3SO_3H$) |
| 8 | n-$C_3H_7$ | H | $CH_3$ | $CH_3$ | | 243 ($CH_3SO_3H$) |
| 9 | $C_6H_5-CH_2$ | H | $CH_3$ | $CH_3$ | | 284 (HCl) |

Beispiel 3

1-/1(4-Amino-6,7-dimethoxi-2-chinazolinyl)-4-piperidyl7-5-isopropyl-2,4-oxazolidindion-methansulfonat

Ein Gemisch aus 0,38 g (0,001 mol) 2-(4-Ethylcarbamido-piperidyl)-4-amino-6,7-dimethoxi-chinazolin, 0,13 g (0,001 mol) 2-Hydroxi-3-methyl-buttersäure-methylester, 2 ml Diglym und 0,04 g (0,001 mol) Natriumhydrid-Dispersion (57 %ig) wird 1 Stunde bei 120°C Innentemperatur gehalten. Nach dem Abkühlen gibt man Eiswasser zu und schüttelt mit Essigester aus. Die Essigester-Phase wäscht man mit Wasser, trocknet und dampft sie ein. Aus dem öligen Rückstand, der 0,6 g beträgt, wird das Methansulfonat hergestellt, das nach dem Umkristallisieren bei 272°C schmilzt.

Analog Beispiel 3 werden die folgenden Substanzen der Formel I erhalten:

Tabelle III

| Nr. | $R_1$ | $R_2$ | $R_3$ | $R_4$ | Fp [° C] Base | Salz |
|---|---|---|---|---|---|---|
| 1 | $CH_3$ | $CH_3$ | $CH_3$ | $CH_3$ | 223 | 293 ($CH_3SO_3$-H) |
| 2 | $CH_3$ | H | $CH_3$ | $CH_3$ | | 280 (HCl) |
| 3 | $CH_3$ | $C_2H_5$ | $CH_3$ | $CH_3$ | 135 | 288 ($CH_3SO_3H$) |
| 4 | n-$C_4H_9$ | H | $CH_3$ | $CH_3$ | 80 | 230 ($CH_3SO_3H$) |
| 5 | $-(CH_2)_4-$ | | $CH_3$ | $CH_3$ | 140 | 295 ($CH_3SO_3H$) |
| 6 | H | H | $CH_3$ | $CH_3$ | | 307 ($CH_3SO_3H$) |
| 7 | $C_2H_5$ | H | $CH_3$ | $CH_3$ | | 298 ($CH_3SO_3H$) |
| 8 | n-$C_3H_7$ | H | $CH_3$ | $CH_3$ | | 243 ($CH_3SO_3H$) |
| 9 | $C_6H_5-CH_2$ | H | $CH_3$ | $CH_3$ | | 284 (HCl) |

Beispiel 4

a) 3-/1-(2-Nitrilo-4,5-dimethoxi-phenyl-thiocarbamido)-4-piperidyl7-5-isopropyl-2,4-oxazolidindion

Zu einer Lösung aus 5,8 g (0,0256 mol) 4-(5-Isopropyl-2,4-oxazolidindion-3-yl)-piperidin in 33 ml Essigester wird bei 0 - 5°C eine Lösung aus 5,6 g (0,0254 mol) 3,4-Dimethoxi-6-isothiocyanato-benzonitril in 32 ml Essigester in 20 Minuten zugetropft. Das Gemisch läßt man 3 Stunden bei 0°C, 13 Stunden bei Raumtemperatur reagieren und saugt ab.
Ausbeute 10,0 g (88,2 % d.Th.), hellbraune Kristalle, Fp. 201°C.

b) 3-/1-(2-Nitrilo-4,5-dimethoxo-phenyl-methyl-thioformamido)-4-piperidyl7-5-isopropyl-2,4-oxazolidindion

Zu einem gerührten Gemisch aus 10,0 g (0,0224 mol) der nach a) erhaltenen Verbindung in 90 ml Essigester werden 2,8 ml (0,0448 mol) Methyljodid gegeben. Nach 5 Stunden Rühren bei 60°C läßt man das Gemisch über Nacht stehen. Nach dem Ausschütteln mit 2 N Natronlauge wäscht man die Essigester-Phase zweimal mit Wasser, trocknet sie und dampft sie ein.
Ausbeute 7,9 g (76,6 % d.Th.); dunkelrotes Öl.

c) 1-/1-(4-Amino-6,7-dimethoxi-2-chinazolinyl)-4-piperidyl7-5-isopropyl-2,4-oxazolidindion-methansulfonat

3,5 g (0,0076 mol) der nach b) erhaltenen Verbindung, 8,0 g (0,15 mol) Ammoniumchlorid und 35 ml Formamid werden unter Stickstoff-Atmosphäre bei 120°C 2 Stunden gerührt. Das Produkt läßt sich, nach dem Versetzen mit Eiswasser, absaugen. Zur Reinigung werden die Kristalle in Essigester gelöst und die Lösung nacheinander mit konz. Ammoniakwasser und zweimal mit Wasser ausgeschüttelt, getrocknet und eingedampft.

Die Kristalle (Fp. 125°C) lößt man in Ethanol, versetzt sie mit Methansulfonsäure und saugt ab.
Ausbeute 1,8 g, Kristalle, Fp. 272°C.

Analog Beispiel 4 werden die folgenden Substanzen der Formel I erhalten:

Tabelle IV

| Nr. | $R_1$ | $R_2$ | $R_3$ | $R_4$ | Fp [° C] Base | Salz |
|---|---|---|---|---|---|---|
| 1 | $CH_3$ | $CH_3$ | $CH_3$ | $CH_3$ | 223 | 293 ($CH_3SO_3$-H) |
| 2 | $CH_3$ | H | $CH_3$ | $CH_3$ | | 280 (HCl) |
| 3 | $CH_3$ | $C_2H_5$ | $CH_3$ | $CH_3$ | 135 | 288 ($CH_3SO_3H$) |
| 4 | n-$C_4H_9$ | H | $CH_3$ | $CH_3$ | 80 | 230 ($CH_3SO_3H$) |
| 5 | $-(CH_2)_4-$ | | $CH_3$ | $CH_3$ | 140 | 295 ($CH_3SO_3H$) |
| 6 | H | H | $CH_3$ | $CH_3$ | | 307 ($CH_3SO_3H$) |
| 7 | $C_2H_5$ | H | $CH_3$ | $CH_3$ | | 298 ($CH_3SO_3H$) |
| 8 | n-$C_3H_7$ | H | $CH_3$ | $CH_3$ | | 243 ($CH_3SO_3H$) |
| 9 | $C_6H_5-CH_2$ | H | $CH_3$ | $CH_3$ | | 284 (HCl) |

Patentansprüche

1. Verbindungen der Formel

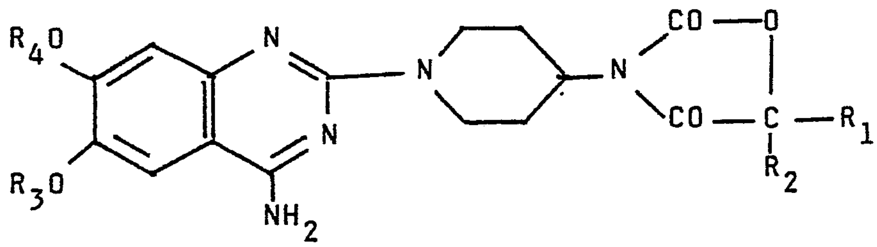

(I)

in der

$R_1$ und $R_2$, die gleich oder verschieden sein können, für Wasserstoff, $C_1$-$C_8$-Alkyl oder Arylmethyl, $R_1$ und $R_2$ gemeinsam auch für $-(CH_2)_4-$ oder $-(CH_2)_5-$,

$R_3$ und $R_4$, die gleich oder verschieden sein können, für Wasserstoff, $C_1$-$C_4$-Alkyl oder Arylmethyl, $R_3$ und $R_4$ gemeinsam auch für $-CH_2-$ oder $-CH_2-CH_2-$ stehen können, gegebenenfalls als Racemate bzw. Stereoisomerengemische oder reine Enentiomere, in Form der freien Basen und der Säureadditionssalze.

2. Verbindungen der Formel I, worin $R_1$ und $R_2$ Wasserstoff oder $C_1$-$C_4$-Alkyl, $R_3$ und $R_4$ Methyl bedeuten.

3. Verfahren zur Herstellung von Verbindungen nach Anspruch 1 oder 2, dadurch gekennzeichnet, daß man a) ein 4-Aminochinoxalin der Formel

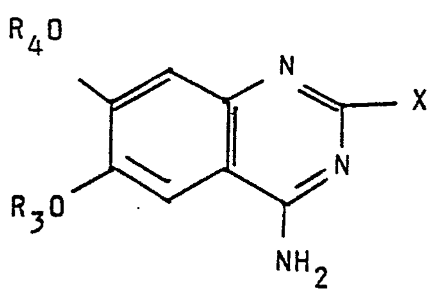

(II),

worin X für Chlor, Brom oder $C_1$-$C_4$-Alkylthio steht und $R_3$ und $R_4$ die obige Bedeutung haben, mit einem Piperidinderivat der Formel

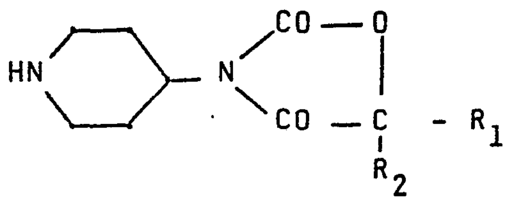

(III)

worin $R_1$ und $R_2$ die obige Bedeutung haben, umsetzt oder daß man

b) eine Verbindung der Formel

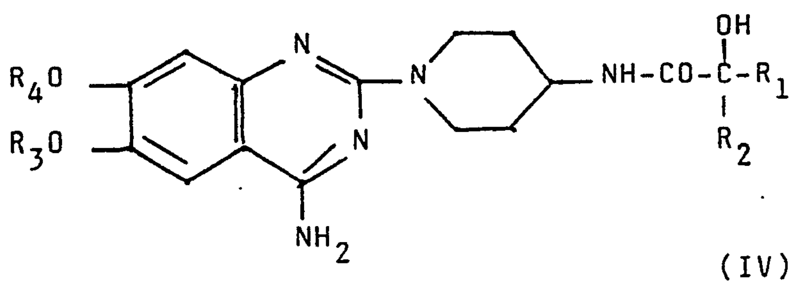

(IV),

$R_1$, $R_2$, $R_3$ und $R_4$ die obige Bedeutung haben, mit einem Dialkylcarbonat der Formel

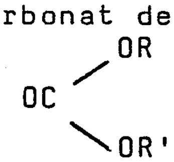

(V),

worin R und R', die gleich oder verschieden sein können, für ggf. verzweigte $C_1$-$C_6$-Alkylreste stehen, umsetzt

oder daß man

c) eine Verbindung der Formel

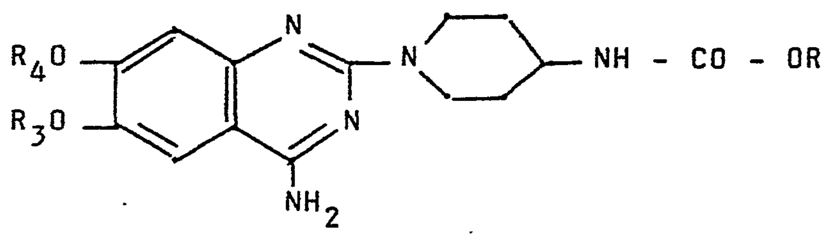

(VI),

worin R, $R_3$ und $R_4$ die obige Bedeutung haben, mit einem Hydroxicarbonsäureester der Formel

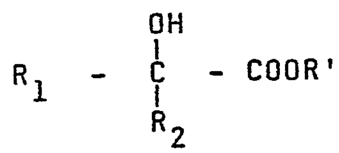

(VII),

worin R', $R_1$ und $R_2$ die obige Bedeutung haben, umsetzt

oder daß man

d) eine Verbindung der Formel

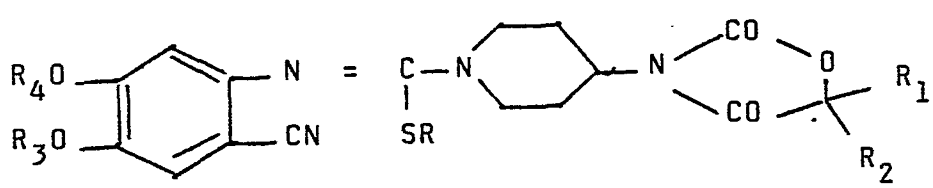

(VIII),

worin R, $R_1$, $R_2$, $R_3$ und $R_4$ die obige Bedeutung haben, mit Ammoniumchlorid und Formamid umsetzt und die nach a) bis d) erhaltenen Verbindungen in Form von Basen oder von Säureadditionssalzen isoliert.

4. Pharmazeutische Zubereitungen, dadurch gekennzeichnet, daß sie neben üblichen Hilfs- und/oder Trägerstoffen als Wirkstoff eine Verbindung nach Anspruch 1 oder 2 enthalten.

5. Verwendung von Verbindungen nach Anspruch 1 oder 2 bei der Behandlung von Herz/Kreislauferkrankungen.